# EUROPÄISCHE PATENTANMELDUNG

(11) **EP 4 745 400 A1**
(43) Veröffentlichungstag der Anmeldung: **20.05.2026**
(21) Anmeldenummer: 24213020.1
(22) Anmeldetag: 14.11.2024
(51) Int. Cl.: F04B 43/12, F04B 53/22

(54) **MEDIZINISCHES SCHLAUCHPUMPEN-AGGREGAT**

(71) Anmelder: Richard Wolf GmbH, 75438 Knittlingen (DE)
(72) Erfinder: Steinhauser, Luis, 75438 Knittlingen (DE); Tewes, Moritz, 75438 Knittlingen (DE); Knoth, Stefan, 75438 Knittlingen (DE); Lampert, Alexander, 75248 Ölbronn-Dürrn (DE)
(74) Vertreter: Patentanwälte Hemmer Lindfeld Frese Partnerschaft mbB

(57) **Zusammenfassung**

Die Erfindung betrifft ein medizinisches Schlauchpumpen-Aggregat mit einem Motorgehäuse (2), welches an seiner Außenseite ein Pumprad (6) aufweist, sowie mit zumindest einer austauschbaren Schlauchkassette (20), in deren Inneren zumindest ein Pumpschlauch (30) angeordnet ist, wobei das Motorgehäuse (2) an der Außenseite mit dem Pumprad (6) eine Kassettenaufnahme (10) zur Aufnahme der Schlauchkassette (20) aufweist, welche dazu ausgebildet ist, die Schlauchkassette (20) lösbar an dem Motorgehäuse (2) zu fixieren, und wobei die Schlauchkassette (20) und die Kassettenaufnahme (10) derart ausgestaltet sind, dass, wenn die Schlauchkassette (20) an dem Motorgehäuse (2) fixiert ist, der zumindest eine Pumpschlauch (30) mit dem Pumprad (6) in Anlage ist.

## Beschreibung

Die Erfindung betrifft ein medizinisches Schlauchpumpen-Aggregat.

Medizinische Schlauchpumpen-Aggregate werden beispielsweise in Verbindung mit endoskopischen Operationssystemen zum Absaugen von Flüssigkeit aus dem Operationsgebiet verwendet. Bei derartigen Peristaltikpumpen ist ein Pumpschlauch um ein Pumprad geführt, welches an seinem Außenumfang Verdrängerkörper aufweist, die bei Rotation des Pumpenrades den Pumpschlauch umlaufend komprimieren und entspannen, wodurch eine Pump- bzw. Förderbewegung im Inneren des Pumpschlauches verursacht wird.

Aus US 8,491,285 B2 ist eine Peristaltikpumpe bekannt, bei welcher ein Gehäuse eine Kassettenaufnahme aufweist, in deren Inneren das Pumprad gelegen ist. Der Pumpschlauch ist in einer Kassette angeordnet, welche in die Kassettenaufnahme eingesteckt wird, wodurch der Schlauch in eine das Pumprad umgreifende Position gebracht wird. Der eigentliche Pumpschlauch ist dabei bogenförmig ausgebildet und endet am offenen Ende der Pumpkassette in zwei Anschlussstücken, von welchen ausgehend sich Anschlussschläuche zum entgegengesetzten Ende der Kassette und dann aus dieser heraus erstrecken. Wenn die Pumpkassette in die Pumpaufnahme eingesetzt ist, liegen die Anschlussstücke am Boden der Kassettenaufnahme. Nachteilig bei dieser Ausgestaltung ist, dass die Kassettenaufnahme schwer zu reinigen ist und die Ausgestaltung der Schlauchkassette in den Anschlussstücken eine starke Strömungsumlenkung erfordert.

Vor diesem Hintergrund ist es Aufgabe der Erfindung, ein medizinisches Schlauchpumpen-Aggregat bereitzustellen, welches eine einfache Reinigung und eine optimierte Strömungsführung durch die Pumpe ermöglicht. Diese Aufgabe wird durch ein medizinisches Schlauchpumpen-Aggregat mit den in Anspruch 1 angegebenen Merkmalen gelöst. Bevorzugte Ausgestaltungen ergeben sich aus den zugehörigen Unteransprüchen, der nachfolgenden Beschreibung sowie den beigefügten Figuren.

Das erfindungsgemäße medizinische Schlauchpumpen-Aggregat weist ein Motorgehäuse auf. Im Inneren des Motorgehäuses sind zumindest ein elektrischer Antriebsmotor und vorzugsweise die erforderliche Steuereinrichtung zum Betrieb des Pumpenaggregates und gegebenenfalls zur Kommunikation mit weiteren Geräten angeordnet. Darüber hinaus können an der Außenseite des Motorgehäuses Bedienelemente zum Einstellen und Bedienen des Schlauchpumpen-Aggregates angeordnet sein. Das Motorgehäuse könnte auch noch weitere Bestandteile eines Operationssystems beinhalten. An seiner Außenseite weist das Schlauchpumpen-Aggregat ein Pumprad auf, welches durch einen im Inneren des Motorgehäuses angeordneten Antriebsmotor drehend antreibbar ist bzw. angetrieben wird. Zur Anordnung an dem Pumprad vorgesehen weist das Schlauchpumpen-Aggregat ferner zumindest eine austauchbare Schlauchkassette auf, in deren Inneren zumindest ein Pumpschlauch angeordnet ist. Das Motorgehäuse weist an der Außenseite, an der das Pumprad gelegen ist, eine Kassettenaufnahme auf, in welcher die Schlauchkassette aufgenommen und gehalten werden kann. Dabei ist die Kassettenaufnahme derart ausgebildet, dass die Schlauchkassette lösbar in der Kassettenaufnahme außen an dem Motorgehäuse fixiert werden kann. Wesentlich ist, dass erfindungsgemäß die Kassettenaufnahme an einer Außenseite des Motorgehäuses und nicht als Ausnehmung im Inneren ausgebildet ist. Dadurch wird zum einen erreicht, dass die Kassettenaufnahme gut zugänglich ist und von außen leicht gereinigt werden kann. Zum anderen steht an der Außenseite des Motorgehäuses mehr Raum zum Zu- und Abführen erforderlicher Schläuche zur Verfügung, so dass die Schläuche mit weniger Bögen oder Abwinklungen verlaufen können und eine günstigere Strömungsführung erreicht werden kann. Die Schlauchkassette und die Kassettenaufnahme sind derart ausgestaltet, dass, wenn die Schlauchkassette an dem Motorgehäuse fixiert ist, das heißt in der Kassettenaufnahme aufgenommen ist, der zumindest eine Pumpschlauch mit dem Pumprad in Anlage ist, so dass an dem Pumprad angeordnete Verdrängerkörper auf den Pumpschlauch einwirken können. Auf diese Weise kann der Pumpschlauch sehr leicht relativ zu dem Pumprad definiert positioniert werden, da er einfach gemeinsam mit der gesamten Schlauchkassette ausgetauscht und an dem Pumprad angeordnet werden kann. Die Lage des Pumpschlauches relativ zu dem Pumprad wird dabei durch die Schlauchkassette und die sie aufnehmende Kassettenaufnahme definiert und vorgegeben, so dass stets eine korrekte Positionierung des Pumpschlauches relativ zu dem Pumprad sichergestellt werden kann, wenn die Schlauchkassette in die Kassettenaufnahme eingesetzt ist. Dadurch kann eine hohe Betriebssicherheit bei leichter Handhabung realisiert werden.

Die Kassettenaufnahme weist bevorzugt zumindest ein Halteelement auf, welches mit einem Abschnitt der Schlauchkassette formschlüssig in Eingriff ist, wenn die Schlauchkassette in die Kassettenaufnahme eingesetzt ist. Das Halteelement ist vorzugsweise so angeordnet, dass es einen Abschnitt der Schlauchkassette umgreift bzw. übergreift, um diese formschlüssig zu fixieren. Weiter bevorzugt ist dabei zumindest ein lösbares Halteelement vorgesehen, welches so angeordnet ist, dass es die Schlauchkassette gegen Entnehmen aus der Kassettenaufnahme fixiert und zum Entnehmen der Schlauchkassette gelöst werden kann. Dies kann beispielsweise ein federbelastetes Rast- oder Eingriffselement sein. Es könnten jedoch auch andere geeignete lösbare Halteelemente zum Einsatz kommen. Beispielsweise könnte auch ein feststehendes Halteelement an der Kassettenaufnahme vorgesehen sein, welches durch ein lösbares Element an der Schlauchkassette um- oder hintergriffen wird. So könnte beispielsweise an der Schlauchkassette eine elastische Zunge oder Wandung ausgebildet sein, welche den Vorsprung an der Kassettenaufnahme lösbar hintergreift.

In einerweiteren möglichen Ausgestaltung weist die Kassettenaufnahme zumindest zwei voneinander beabstandete Halteelemente auf. Ferner weist bei dieser Ausgestaltung die Schlauchkassette vorzugsweise an zwei voneinander abgewandten Seiten Eingriffsabschnitte auf, welche jeweils mit einem der Halteelemente in Eingriff sind, wenn die Schlauchkassette in die Kassettenaufnahme eingesetzt ist. Die Halteelemente sind vorzugsweise so angeordnet, dass sie bezogen auf die Einsetzrichtung der Schlauchkassette seitlich an der Schlauchkassette angreifen bzw. seitlich an der Schlauchkassette vorgesehene Eingriffsabschnitte umgreifen. Die Eingriffsabschnitte können dabei vorzugsweise zwischen den Halteelementen und einer Außenwandung des Motorgehäuses aufgenommen werden, so dass die Schlauchkassette an dieser Außenwandung anliegt. Die Halteelemente sind weiter bevorzugt so angeordnet, dass beim Einsetzen der Schlauchkassette in die Kassettenaufnahme die Eingriffsabschnitte und die Halteelementen ineinandergreifen und gegebenenfalls um ein gewisses Einschubmaß entlang der Halteelemente bewegt werden. So können die Halteelemente beispielsweise als Nut oder als Vorsprung ausgebildet sein, welcher einen stegförmigen Eingriffsabschnitt übergreift. So können die Halteelemente gleichzeitig zur Führung der Schlauchkassette in der Einsetzrichtung dienen. In einer weiteren möglichen Ausgestaltung könnten die Eingriffsabschnitte jeweils als Nut oder Vorsprung ausgebildet sein. Die Halteelemente sind bevorzugt so angeordnet, dass sie die Eingriffsabschnitte so umgreifen bzw. hintergreifen, dass durch sie die Schlauchkassette in einer Richtung quer, besonders bevorzugt senkrecht zu der Außenseite des Motorgehäuses gehalten bzw. gesichert wird.

In einer weiteren möglichen Ausgestaltung weist die zumindest eine Schlauchkassette ein in Einsetzrichtung vorderes Ende auf, welches derart offen ausgebildet ist, dass die Schlauchkassette beim Einsetzen in die Kassettenaufnahme mit dem offenen Ende über das Pumprad geschoben werden und dieses übergreifen kann. Das heißt das Pumprad tritt vom offenen Ende her in die Schlauchkassette ein und kommt so mit dem in der Schlauchkassette geführten Pumpschlauch derart in Anlage, dass es die gewünschte Pumpwirkung erzeugen kann. Bevorzugt wird beim Einsetzen das Pumprad mit seiner Außenumfangsseite auf den in der Schlauchkassette geführten Pumpschlauch zu bewegt. Das heißt der Pumpschlauch wird gemeinsam mit der Schlauchkassette über einen Abschnitt des Außenumfanges des Pumprades geschoben. Das Pumprad steht vorzugsweise von einer Außenseite des Motorgehäuses vor. Die Drehachse des Pumprades erstreckt sich vorzugsweise quer, weiter bevorzugt im Wesentlichen normal bzw. senkrecht zu der Außenseite bzw. Außenwandung des Motorgehäuses. Wenn die Außenseite des Motorgehäuses schräg zur Vertikalen angeordnet ist, erstreckt sich somit auch die Drehachse vorzugsweise schräg. Die Schlauchkassette kommt dabei vorzugsweise zumindest mit einzelnen Wandbereichen an der Außenseite des Motorgehäuses zur Anlage. Besonders bevorzugt weist die Schlauchkassette eine im Wesentlichen U-förmig ausgestaltete Wandstruktur auf, welche sich normal zu der Außenseite des Motorgehäuses erstreckt, wenn die Schlauchkassette in die Kassettenaufnahme eingesetzt ist. Der offene Bereich der U-förmigen Wandungsstruktur zwischen den zwei seitlichen Schenkeln bildet dabei das beschriebene offen ausgebildete vordere Ende der Schlauchkassette.

Gemäß einer weiteren möglichen Ausgestaltung weist die zumindest eine Schlauchkassette eine dem Motorgehäuse bzw. dessen Außenwandung zugewandte offene Seite auf, durch welche sich das Pumprad in das Innere der Schlauchkassette erstreckt, wenn die Schlauchkassette in die Kassettenaufnahme eingesetzt ist. Die offene Seite wird dabei vorzugsweise von der Stirnkante einer U-förmigen Wandstruktur begrenzt, wie sie vorangehend beschrieben wurde. Mit der Stirnkante liegt die U-förmige Wandstruktur in der Kassettenaufnahme vorzugsweise so, dass sie der Außenseite des Motorgehäuses zugewandt ist, besonders bevorzugt an die Außenseite bzw. Außenwandung des Motorgehäuses angrenzt. An der entgegengesetzten Seite, welche der Oberfläche des Motorgehäuses abgewandt ist, erstreckt sich vorzugsweise eine geschlossene Seitenfläche der Schlauchkassette quer zu der U-förmigen Wandstruktur. Bei dieser Ausgestaltung weist die Schlauchkassette somit eine geschlossene Seitenwandung auf, welche dem Motorgehäuse abgewandt ist, wenn die Schlauchkassette in die Kassettenaufnahme eingesetzt ist. Mit einer offenen Seite grenzt die Schlauchkassette dabei an das Motorgehäuse an bzw. liegt an dem Motorgehäuse an. So kann das Pumprad durch die offene Seite in das Innere der Schlauchkassette eingreifen, um mit dem zumindest einen Pumpschlauch in Anlage zu treten. Die Schlauchkassette kann zum Beispiel quer und/oder parallel zur Außenseite des Motorgehäuses über das Pumprad geschoben werden. Quer zu diesen Seiten erstreckt sich eine Umfangswandung, vorzugsweise in Form der beschriebenen U-förmigen Wandstruktur, wobei eine in Einsetzrichtung vordere Seite ebenfalls offen ausgebildet ist, so dass die Schlauchkassette über das Pumprad z.B. in einer Einsetzrichtung parallel zur Außenseite des Motorgehäuses aufgeschoben werden kann.

Wesentlicher Vorteil der erfindungsgemäßen Ausgestaltung und der beschriebenen bevorzugten Ausgestaltungen ist, dass das Pumprad an der Außenseite des Motorgehäuses frei zugänglich angeordnet ist, und so leicht gereinigt und gewartet werden kann. Im Betrieb wird es vorzugsweise vollständig von der Schlauchkassette überdeckt, so dass der Bereich zwischen Pumprad und Pumpschlauch abgedeckt ist, so dass in diesen Bereich beim Betrieb keine Fremdkörper oder Gegenstände eintreten können. So wird die Betriebssicherheit verbessert und gleichzeitig ein Verletzungsrisiko für Bedienpersonen minimiert, da die beweglichen Teile im Betrieb abgedeckt sind.

Gemäß einer weiteren möglichen Ausführungsform der Erfindung weist die zumindest eine Schlauchkassette eine Trägerstruktur aus Kunststoff auf, in welcher der zumindest eine Pumpschlauch an seinen Axialenden gehalten ist. Dies ermöglicht es, den Pumpschlauch mit definierter Länge so in der Schlauchkassette zu fixieren, dass, wenn die Schlauchkassette in die Kassettenaufnahme eingesetzt ist, der Pumpschlauch das Pumprad in einer definierten Weise umschlingt und mit einer gewünschten Anlagekraft an dem Pumprad anliegt. So wird verhindert, dass der Pumpschlauch im Bereich des Pumprades zu locker oder zu stramm angeordnet ist. Auf diese Weise kann eine hohe Betriebssicherheit bei einfacher Handhabung realisiert werden.

Gemäß einer weiteren bevorzugten Ausgestaltung weist die Schlauchkassette eine bogenförmige Führung und/oder Anlagefläche für den zumindest einen Pumpschlauch auf. Diese Anlagefläche ist vorzugsweise dem Außenumfang des Pumprades zugewandt, wenn die Schlauchkassette in die Kassettenaufnahme eingesetzt ist. Dabei ist die Anlagefläche in radialer Richtung bezogen auf die Drehachse des Pumprades um ein definiertes Maß vom Außenumfang des Pumprades beabstandet. Die Anlagefläche bildet ein Wider- bzw. Gegenlager für den Pumpschlauch, so dass der Pumpschlauch durch die Verdrängerkörper an dem Pumpenrad gegen die Anlagefläche gedrückt werden kann. Dabei kann der zumindest eine Pumpschlauch durch die Verdrängerkörper abschnittsweise zugedrückt werden. Durch dieses Wiederlager bzw. die Anlagefläche wird ein zuverlässiger Unterdruckaufbau im Inneren des Pumpschlauches bei Drehung des Pumprades gewährleistet.

Die Schlauchkassette weist weiter bevorzugt zumindest ein, vorzugsweise zwei Anschlusselemente auf, welche den zumindest einen Pumpschlauch mit einem Eingangsschlauch und/oder einem Austrittsschlauch verbinden. Bevorzugt weist die Schlauchkassette somit zwei zueinander beabstandet angeordnete Anschlusselemente auf, zwischen denen sich der zumindest eine Pumpschlauch bogenförmig erstreckt. An einem Anschlusselement ist ein Eingangsschlauch und an dem anderen Anschlusselement ein Austrittsschlauch angeschlossen. Diese Anordnung ermöglicht es, den Pumpschlauch in definierter Länge zwischen den Anschlusselementen anzuordnen und gegebenenfalls den Pumpschlauch in Durchmesser und Material anders zu konfigurieren als die sich anschließenden Schläuche.

Die beschriebenen Anschlusselemente sind vorzugsweise aus Kunststoff gefertigt und weiter bevorzugt einstückig mit der Trägerstruktur der Schlauchkassette ausgebildet oder in der Trägerstruktur kraft-, formschlüssig und/oder stoffschlüssig fixiert. So kann kostengünstig ein einteiliger Aufbau der Trägerstruktur mit den Anschlusselementen geschaffen werden und die Anschlusselemente können an definierten Stellen in der Struktur der Schlauchkassette die Enden des Pumpschlauches fixieren.

Vorzugsweise ist die Schlauchkassette mit dem zumindest einen Pumpschlauch als austauschbarer zum Einmalgebrauch vorgesehener Artikel ausgebildet. Auch die sich anschließenden Eingangs- und Austrittsschläuche können mit der Schlauchkassette als vormontierter Artikel zum Einmalgebrauch bereitgestellt werden. Dies ermöglicht eine einfache Handhabung, da nach dem Betrieb die gesamte Schlauchkassette von dem Motorgehäuse gelöst werden und entsorgt werden kann und für die nächste Operation durch eine neue vormontierte Schlauchkassette ersetzt werden kann. Dies minimiert den Reinigungsaufwand.

Gemäß einer speziellen Ausführungsform der Erfindung kann die Schlauchkassette in ihrem Inneren mehrere, besonders bevorzugt zwei parallele Pumpschläuche aufweisen. Das Pumprad ist dabei so ausgebildet, dass es mit beiden parallelen Pumpschläuchen in Anlage ist, wenn die Schlauchkassette in die Kassettenaufnahme eingesetzt ist, so dass die Verdrängerkörper an dem Pumprad auf beide Pumpschläuche einwirken. Durch die Anordnung zweier Pumpschläuche kann ein gleichmäßigeres Pumpen erreicht werden. Ferner kann ein größerer Strömungsquerschnitt in der Pumpe bereitgestellt werden.

Bei der Anordnung von zwei Pumpschläuchen weist das Pumprad weiter bevorzugt zumindest zwei Verdrängerkörper auf, die in axialer Richtung versetzt bzw. beabstandet zueinander angeordnet sind. So wirkt von diesen zumindest zwei Verdrängerkörpern zumindest ein erster Verdrängerkörprer auf einem ersten der beiden parallelen Pumpschlöuche ein und es wirkt zumindest ein zweiter Verdrängerkörper auf einen zweiten der beiden parallelen Pumpschläuche ein. Es können auch mehrere erste und mehrere zweite Verdrängerkörper über den Umfang des Pumprades verteilt, insbesondere gleichmäßig verteilt, angeordnet sein. Dabei sind die ersten Verdrängerkörper vorzugsweise in einer ersten Ebene quer zur Drehachse des Pumprades und die zweiten Verdrängerkörper in einer zweiten Ebene quer zur Drehachse des Pumprades angeordnet, wobei die erste und die zweite Ebene in Richtung der Drehachse versetzt sind, insbesondere parallel zueinander versetzt sind. So sind vorzugsweise eigene Verdrängerkörper für den ersten und für den zweiten Pumpschlauch vorgesehen. Dies ermöglicht es gemäß einer weiteren möglichen Ausgestaltung der Erfindung, dass der zumindest eine erste und der zumindest eine zweite Verdrängerkörper in Drehrichtung versetzt zueinander an dem Pumprad angeordnet sind. So wird bei Drehung des Pumprades erreicht, dass abwechselnd ein Unterdruck in dem ersten Pumpschlauch und in dem zweiten Pumpschlauch aufgebaut wird, so dass insgesamt ein gleichmäßigerer Fluss durch die Pumpe erreicht wird bzw. die sich durch den Umlauf der Verdrängerkörper ergebenden Pulsationen in dem ersten und dem zweiten Pumpschlauch zumindest teilweise kompensiert werden. So wird insgesamt die Pulsation der Pumpe verringert.

Gemäß einer weiteren möglichen Ausgestaltung der Erfindung weisen die Anschlusselemente jeweils zumindest einen, vorzugsweise zwei, Pumpschlauchstutzen zum Anschluss jeweils eines Pumpschlauches auf, die sich vorzugsweise quer oder tangential zur Drehachse des Pumprades erstrecken. Bei einer Ausführungsform mit zwei parallel verlaufenden Pumpschläuchen können bevorzugt zwei Pumpschlauchstutzen vorgesehen sein. Dazu sind die Pumpschlauchstutzen an jedem Anschlusselement vorzugsweise parallel zueinander und in Richtung der Drehachse des Pumprades parallel versetzt zueinander angeordnet. Neben einem oder zwei Pumpschlauchstutzen weist jedes Anschlusselement vorzugsweise einen Anschlussstutzen auf, welcher, je nach Verwendung des Anschlusselementes zum Anschluss eines Eingangs- oder Austrittsschlauches vorgesehen ist. Der Anschlussstutzen und der oder die Pumpschlauchstutzen erstrecken sich von dem Anschlusselement vorzugsweise in entgegengesetzten Richtungen, d.h. insbesondere diametral entgegengesetzt zu einer Längsachse des Anschlusselementes. So wird erreicht, dass ein an dem Anschlussstutzen angeschlossener Schlauch, beispielsweise ein Eingangs- oder Austrittsschlauch, sich im Wesentlichen in Verlängerung des Pumpschlauches erstrecken kann, so dass in dem Anschlusselement vorzugsweise nur eine geringe oder gar keine Strömungsumlenkung erfolgt und der so der Strömungswiederstand minimiert wird.

Gemäß einerweiteren Ausführungsform der Erfindung weist zumindest eines der Anschlusselemente, vorzugsweise dasjenige Anschlusselement, welches zur Verbindung mit einem Austrittsschlauch vorgesehen ist, einen Drucksensor auf oder ist zur Aufnahme eines Drucksensors oder alternativ zur Anlage an einem Drucksensor ausgebildet. Dies ermöglicht über den Drucksensor eine Druckmessung direkt im Anschlusselement. So kann vorzugsweise der Druck an der Austrittsseite gemessen und überwacht werden. Dadurch kann beispielsweise ein zu hoher Druck an der Austrittsseite detektiert werden, um ein Alarmsignal auszugeben und/oder den Betrieb der Pumpe zu stoppen, beispielsweise um ein Platzen eines ausgangsseitig der Pumpe angeordneten Behälters oder Abspringen eines Schlauches aufgrund des Überdruckes zu verhindern. Wenn das Anschlusselement zur Anlage an einem Drucksensor ausgebildet ist, hat dies den Vorteil, dass eine Druckmessung im Inneren des Anschlusselementes möglich ist, ohne dass der Drucksensor selber in der Schlauchkassette angeordnet werden müsste. So kann die Schlauchkassette mit dem Anschlusselement beispielsweise mit einem fest an der Kassettenaufnahme angeordneten Drucksensor zusammenwirken, um den Druck im Inneren des Anschlusselementes bzw. im Strömungsweg im Inneren der Schlauchkassette zu bestimmen.

Gemäß einer weiteren möglichen Ausführungsform weist eines der Anschlusselemente, vorzugsweise dasjenige Anschlusselement, welches zur Verbindung mit einem Austrittsschlauch vorgesehen ist, eine an einen Fluidkanal im Inneren bzw. an den Innenraum des Anschlusselementes angrenzende Membran auf. Eine solche Membran dient der Druckübertragung nach außen. Das heißt diese Membran kann beispielsweise mit einem Drucksensor oder einem anderen geeigneten Sensor zur Druckmessung zusammenwirken. So kann beispielsweise die Membranauslenkung als Größe repräsentativ für den Druck erfasst werden oder die Membran an einem externen Drucksensor zur Anlage kommen. Die Kassettenaufnahme weist dazu vorzugsweise einen Sensor, beispielsweise einen Drucksensor, auf, welcher derart angeordnet und ausgebildet ist, dass er mit der Membran zur Druckmessung zusammenwirkt, wenn die Schlauchkassette in die Kassettenaufnahme eingesetzt ist. Besonders bevorzugt kommt die Membran an dem Sensor in der Kassettenaufnahme zur Anlage, wenn sich die Schlauchkassette in der Betriebsposition befindet.

Die Schlauchkassette weist vorzugsweise an ihrem in Einsetzrichtung vorderen Ende zumindest eine sich quer zur Einsetzrichtung, weiter bevorzugt schräg zur Einsetzrichtung erstreckende Anlagefläche auf. Die Anlagefläche erstreckt sich dabei ferner im Wesentlichen quer oder normal bzw. senkrecht zu der Außenseite des Motorgehäuses, an der die Kassettenaufnahme angeordnet ist. Die schräge Erstreckung verläuft vorzugsweise in einem Winkel zwischen 30 und 60° bezogen auf die Einsetzrichtung. Die Kassettenaufnahme weist bevorzugt zumindest eine Gegenanlagefläche auf, welche so angeordnet ist, dass sie der Anlagefläche der Schlauchkassette gegenüberliegt und vorzugsweise an der Anlagefläche anliegt, wenn die Schlauchkassette in die Kassettenaufnahme eingesetzt ist. Die Anlagefläche und Gegenanlagefläche erstrecken sich vorzugsweise im Wesentlichen parallel zueinander. Die sich quer zur Einsetzrichtung, insbesondere schräg zur Einsetzrichtung erstreckende Anlagefläche ermöglicht es, dass die Schlauchkassette beim Einsetzen in die Kassettenaufnahme mit ihrer Anlagefläche gegen die Gegenanlagefläche gedrückt wird, so dass beide in Anlage gehalten werden. Da die Anlagefläche vorzugsweise im Bereich des Anschlusselementes gelegen ist, hat die gewinkelte bzw. schräge Lage der Anlagefläche den Vorteil, dass sich ein Pumpschlauchstutzen bevorzugt ebenfalls schräg zur Einsetzrichtung weg erstrecken kann, so dass eine günstige Schlauchführung für die sich anschließenden Schläuche in Verlängerung des Pumpschlauches realisiert werden kann. Weiter bevorzugt ist zumindest eine an einen Fluidkanal der Schlauchkassette bzw. an einen Strömungsweg in der Schlauchkassette angrenzende Membran, besonders bevorzugt die oben beschriebene Membran zur Druckmessung, in einer der beschriebenen Anlageflächen gelegen und zumindest ein mit der Membran zusammenwirkender Sensor ist in einer gegenüberliegenden Gegenanlagefläche der Kassettenaufnahme angeordnet. So wird beim Einsetzen der Schlauchkassette in die Kassettenaufnahme die Anlagefläche mit der Membran an der Gegenanlagefläche mit dem Sensor zur Anlage gebracht, so dass von dem Sensor über die Membran eine Druckmessung im Inneren des Fluidkanals erfolgen kann. Wie oben beschrieben ist dies bevorzugt ein Fluidkanal an der Austritts- bzw. Druckseite der Schlauchkassette.

Gemäß einer weiteren möglichen Ausgestaltung der Erfindung kann die Schlauchkassette zumindest ein Datenspeicherelement aufweisen. Dieses kann z.B. Betriebsdaten speichern und/oder Konfigurationsdaten enthalten, auf deren Grundlage die Steuerung des Antriebs erfolgt. Ferner ist es möglich, dass das Datenspeicherelement Identifikationsdaten enthält, welche eine Schlauchkassette identifizieren, so dass durch Auslesen des Datenspeicherelementes eine Steuerung in dem Motorgehäuse z.B. erkennen kann, welche Art von Schlauchkassette in die Kassettenaufnahme eingesetzt ist. Vorzugsweise ist zum Auslesen der Datenspeichereinrichtung eine korrespondierend ausgebildete Leseeinrichtung an einer gegenüberliegenden Gegenanlagefläche angeordnet. Die Leseeinrichtung kann beispielsweise eine Antenneneinrichtung sein, welche zum Auslesen und/oder Schreiben eines RFID-Datenspeicherelementes geeignet ist. Es können jedoch auch andere Lese- oder Schreibeinrichtungen, beispielsweise magnetische, optische oder elektrische Schreib- und/oder Leseeinrichtungen zur Kommunikation mit einer Datenspeichereinrichtung in der Gegenkontaktfläche gelegen sein. Besonders bevorzugt ist die Schlauchkassette so ausgebildet, dass an einem Anschlusselement eine Anlagefläche mit einer Membran, wie sie vorangehend beschrieben wurde, und an der entgegengesetzten Seite, im Bereich eines zweiten Anschlusselementes, an der dort gelegenen Anlagefläche eine Datenspeichereinrichtung gelegen ist. So kann die Paarung einer Anlagefläche mit einer Gegenanlagefläche zur Druckmessung und die Paarung einer zweiten Anlagefläche mit einer zweiten Gegenanlagefläche zur Datenübertragung genutzt werden.

Nachfolgend wird die Erfindung beispielhaft anhand der beigefügten Figuren beschrieben. In diesen zeigt:
- Fig. 1: ein medizinisches Schlauchpumpen-Aggregat gemäß der Erfindung ohne Schlauchkassette,
- Fig. 2: ein medizinisches Schlauchpumpen-Aggregat gemäß Figur 1 mit aufgesetzter Schlauchkassette,
- Fig. 3: eine Frontansicht des Schlauchpumpen-Aggregates gemäß Figur 1,
- Fig. 4: eine Frontansicht des Schlauchpumpen-Aggregates mit Schlauchkassette gemäß Figur 2,
- Fig. 5: eine perspektivische Ansicht einer Rückseite der Schlauchkassette,
- Fig. 6: eine Draufsicht auf die Rückseite der Schlauchkassette gemäß Figur 5,

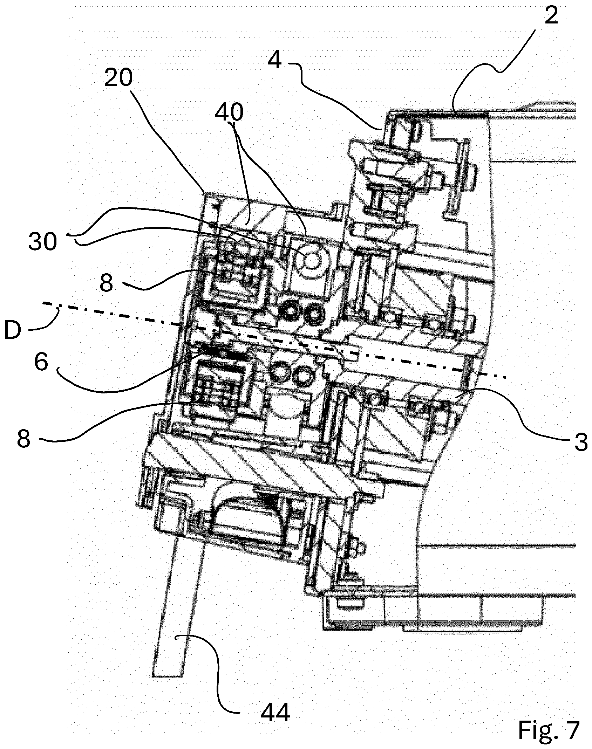

- Fig. 7: eine teilweise geschnittene Ansicht des medizinischen Schlauchpumpen-Aggregates gemäß Figur 2,

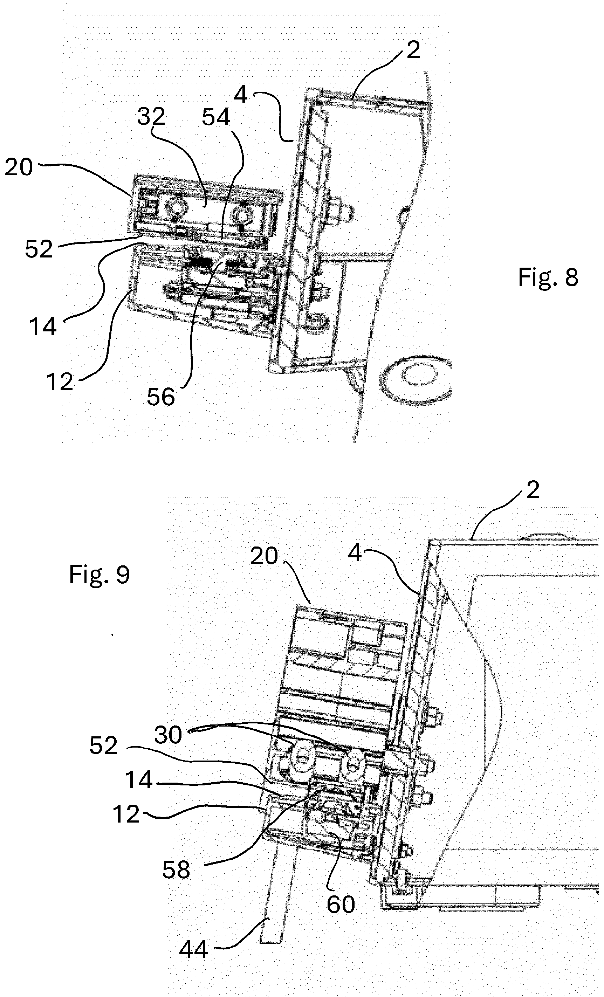

- Fig. 8: eine Schnittansicht durch einen ersten Teil der Anlagefläche und der Gegenanlagefläche sowie
- Fig. 9: einen Schnitt durch einen zweiten Bereich der Anlagefläche mit gegenüberliegender Gegenanlagefläche.

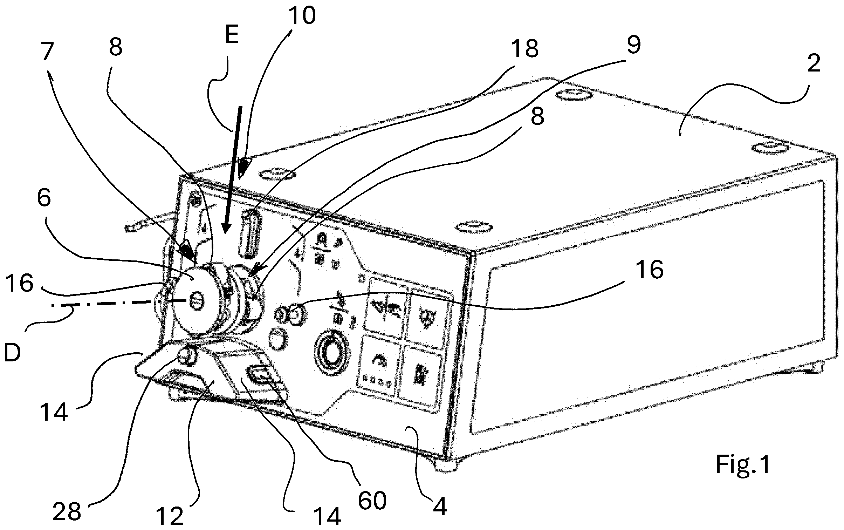

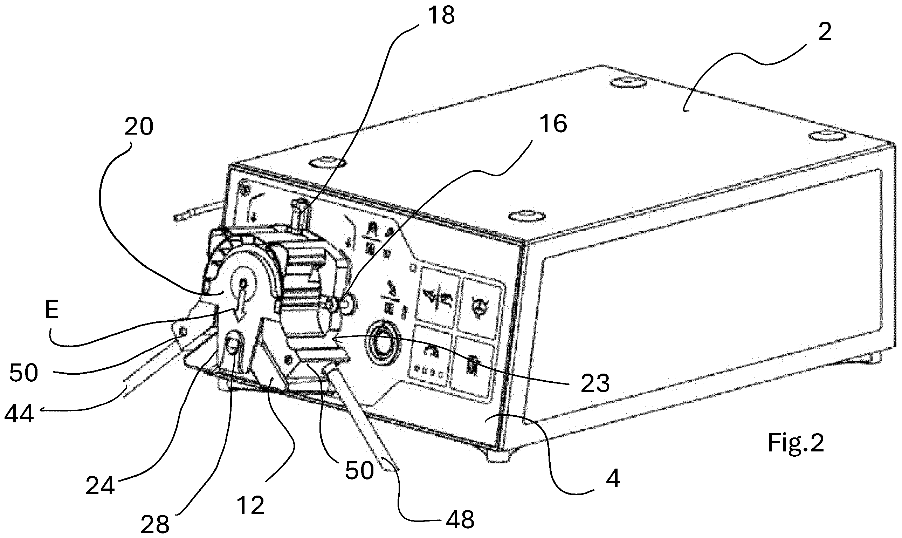

Die Figuren 1 und 2 zeigen den Gesamtaufbau eines erfindungsgemäßen medizinischen Schlauchpumpen-Aggregates. Das medizinische Schlauchpumpen-Aggregat weist ein Motorgehäuse 2 auf, in welchem zumindest ein elektrischer Antriebsmotor, eine Motorsteuerung und ggf. weitere elektronische und elektrische Bauteile zum Betrieb der medizinischen Schlauchpumpe und ggf. weiterer medizinischer Instrumente angeordnet sein können. An einer Außenwandung bzw. Außenseite, in diesem Fall der Frontseite 4 ist ein Pumprad 6 angeordnet, welches über die Welle 3 von dem elektrischen Antriebsmotor im Inneren des Motorgehäuses 2 um die Drehachse D drehend angetrieben werden kann. Das Pumprad weist in diesem Ausführungsbeispiel zwei in Richtung der Drehachse D versetzte Ebenen 7, 9 auf, in welcher jeweils zwei Verdrängerkörper in Form von Verdrängerrollen 8 angeordnet sind. Die Verdrängerrollen 8 in den beiden Ebenen 7, 9 sind um 90° versetzt zueinander angeordnet. In jeder der beiden Ebenen 7, 9 sind die zwei Verdrängerrollen 8 um 180° zueinander beabstandet.

Das Pumprad 6 ist an der Frontseite 4 im Bereich einer Kassettenaufnahme 10 angeordnet. Die Kassettenaufnahme 10 weist unterhalb des Pumprades 6 einen von der Frontseite 4 vorstehenden Anlagekörper 12 auf, welche an seiner Oberseite eine Gegenanlageflächen 14 für eine Schlauchkassette bildet. Seitlich des Pumprades 6 weist die Kassettenaufnahme darüber hinaus zwei von der Frontseite 4 vorstehende Halteelemente 16 auf. Die Halteelemente 16 sind pilzförmig ausgebildet und können mit ihren auskragenden Köpfen seitliche Abschnitte einer Schlauchkassette 20 übergreifen. Oberhalb des Pumprades 6 ist darüber hinaus ein Führungssteg 18, welcher von der Frontseite 4 vorsteht, ausgebildet. Der Führungssteg 18 erstreckt sich parallel zu einer Einsetzrichtung E, in welcher eine Schlauchkassette 20 in die Kassettenaufnahme 10 eingesetzt werden kann.

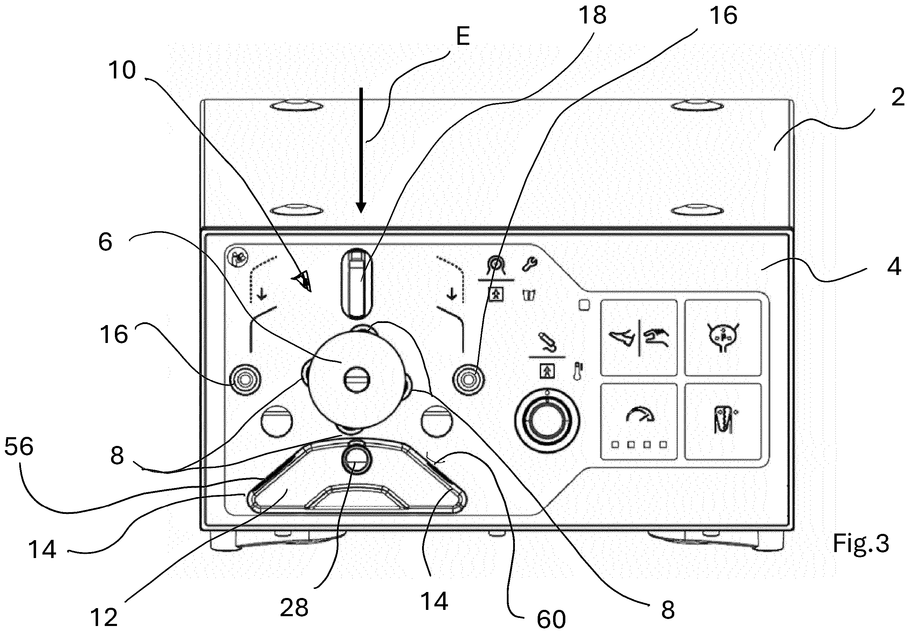

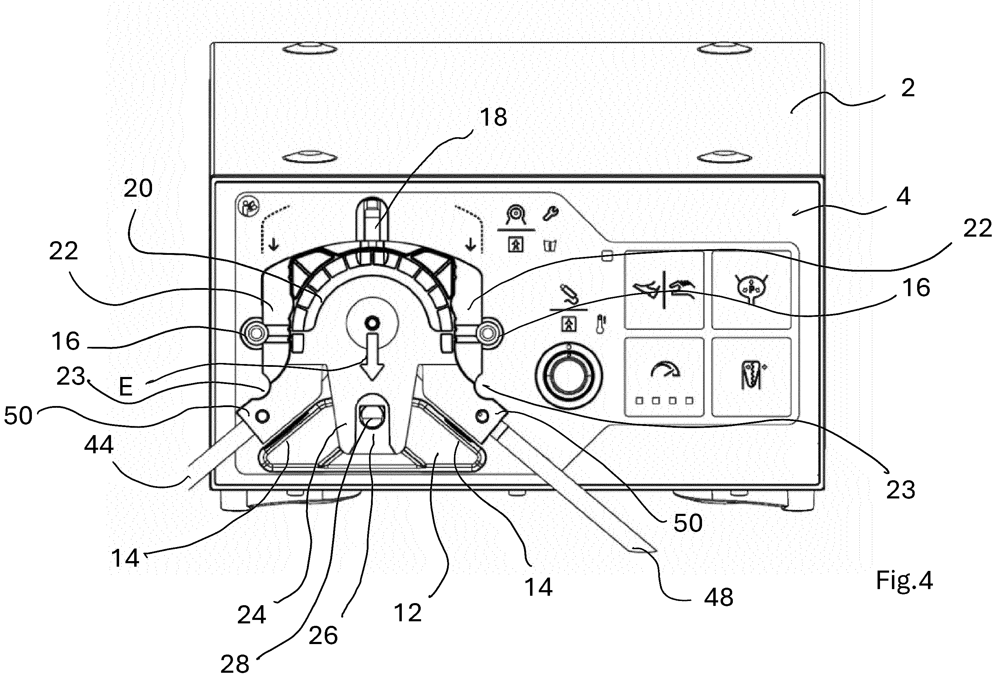

Während die Figuren 1 und 3 das Motorgehäuse 2 ohne die angesetzte Schlauchkassette 20 zeigen, ist in den Figuren 2 und 4 entsprechend das Motorgehäuse 2 mit der angesetzten Schlauchkassette 20 gezeigt. Die Schlauchkassette 20 wird zum Einsetzen in die Kassettenaufnahme zunächst parallel zur Drehachse D und dann in der Einsetzrichtung E in die Kassettenaufnahme 10 eingeschoben. Dabei wird die Schlauchkassette 20 geführt von dem Führungssteg 18 und den Halteelementen 16 in der Einsetzrichtung E parallel zur Oberfläche der Frontseite 4 über das Pumprad 6 geschoben, bis die Schlauchkassette 20 an den Gegenanlageflächen 14 an der Oberseite des Anlagekörpers 12 anliegt. Zum Halten der Schlauchkassette 20 an der Frontseite 4 weist die Schlauchkassette 20 zwei seitliche Eingriffsabschnitte 22 auf. Diese erstrecken sich quer zur Einsetzrichtung E laschen- bzw. rippenförmig nach au-ßen und werden von den Halteelementen 16 übergriffen. Wenn die Schlauchkassette 20 beim Einsetzen zunächst quer zur Frontseite 4 bewegt wird, greifen die Halteelemente unterhalb der Eingriffsabschnitte 22 jeweils in eine Ausnehmung 23 ein. Wenn die Schlauchkassette 20 anschließend in der Einsetzrichtung E parallel zur Fronseite 4 verschoben wird, greifen die Eingriffsabschnitte 22 hinter die pilzförmigen Köpfe der Halteelemente 16. Ferner weist die Schlauchkassette 20 an ihrem in Einsetzrichtung E vorderen Ende eine Lasche 24 mit einer Öffnung 26 auf. In die Öffnung 26 greift, wenn die Schlauchkassette 20 in die Kassettenaufnahme 10 eingesetzt ist, ein federnder Rastvorsprung 28 ein, welcher an dem Anlagekörper 12 gelagert ist. Durch Druck auf den Rastvorsprung 28 kann dieser von der Öffnung 26 außer Eingriff gebracht werden und die Schlauchkassette 20 kann entgegen der Einsetzrichtung E aus der Kassettenaufnahme 10 entnommen werden.

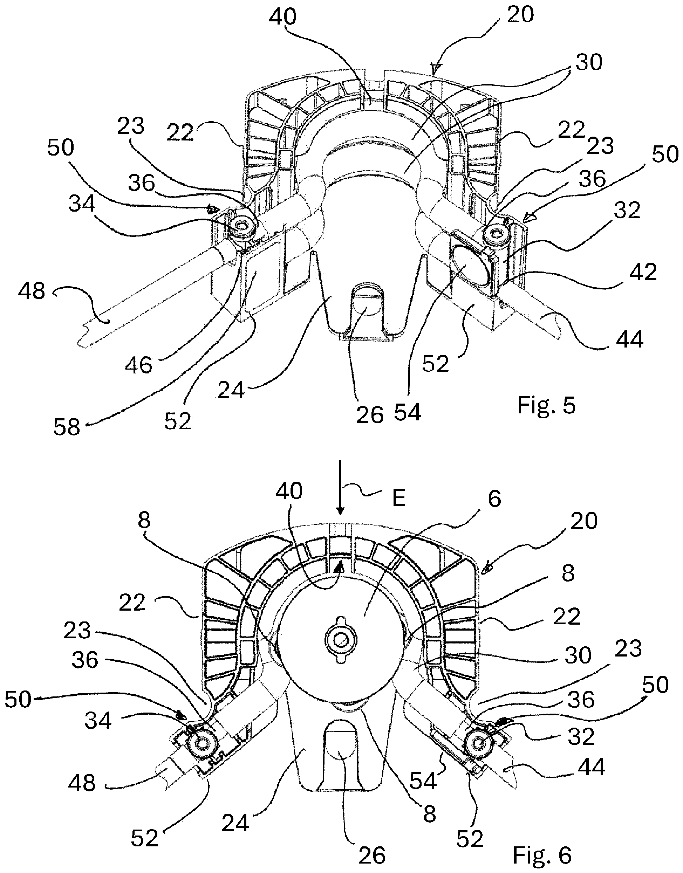

Die Figuren 5 und 6 zeigen Ansichten der Rückseite der Schlauchkassette 20, welche beim Einsetzen in die Kassettenaufnahme 10 der Frontseite 4 zugewandt ist. Die Schlauchkassette 20 ist als formstabile Struktur, vorzugsweise als Kunststoffspritzgussteil ausgebildet. Im Inneren der Schlauchkassette 20 sind zwei parallele Pumpschläuche 30 angeordnet. Die Pumpschläuche 30 sind an ihren Enden jeweils mit einem Anschlusselement 32, 34 verbunden. Die Anschlusselemente 32 und 34 sind hier als Einsätze ausgebildet, welche in die Struktur der Schlauchkassette 20 eingesetzt sind, könnten jedoch auch einstückig mit der weiteren Struktur ausgebildet sein. Jedes der Anschlusselemente 32, 34 weist zwei Pumpschlauchstutzen 36 für die Pumpschläuche 30 auf. Die Pumpschläuche 30 sind mit ihren Enden auf die Pumpschlauchstutzen 36 aufgesteckt. Im Inneren der Schlauchkassette 20 liegen die Pumpschläuche 30 an einer bogenförmigen Anlagefläche 40 an. Die bogenförmige Anlagefläche 40 erstreckt sich konzentrisch zu der Drehachse D, wenn die Schlauchkassette 20 in die Kassettenaufnahme 10 eingesetzt ist. Die Anlagefläche 40 bildet ein Widerlager. Im Pumpbetrieb drücken die Verdrängerrollen 8 die Pumpschläuche 30 gegen die Anlagefläche 40. Insofern ist der Radius der bogenförmigen Anlagefläche 40 so gewählt, dass er einen entsprechend abgestimmten Abstand zu der Drehachse D hat, wenn die Schlauchkassette 20 in die Kassettenaufnahme 10 eingesetzt ist.

Die Anschlusselemente 32, 34 sind im Wesentlichen rohrförmig ausgebildet, wobei sich die Pumpschlauchstutzen 36 jeweils quer zur Längserstreckung der Anschlusselemente 32, 34 erstrecken. Das Anschlusselement 32 weist einen Anschlussstutzen 42 auf, welcher sich bezüglich der Längsachse des Anschlusselementes 32 radial entgegengesetzt zu den Pumpschlauchstutzen 36 erstreckt. Auf den Anschlussstutzen 42 ist ein Austrittsschlauch 44 aufgesetzt. Das Anschlusselement 34 weist entsprechend einen Anschlussstutzen 46 auf, welcher sich bezüglich der Längsachse des Anschlusselementes 34 radial entgegengesetzt zu den Pumpschlauchstutzen 36 erstreckt und auf welchen ein Eingangsschlauch 48 aufgesteckt ist. Die Anschlussstutzen 42 und 46 erstrecken sich in einem spitzen Winkel zur Einsetzrichtung E, in diesem Beispiel in einem Winkel von ungefähr 45°. Dies bewirkt, dass sich der Eingangsschlauch 48 und der Austrittsschlauch 44 im Betrieb schräg nach unten erstrecken. Die Pumpschlauchstutzen 36 erstrecken sich in paralleler Richtung zu den Anschlussstutzen 42 und 46, sodass sich der sich anschließende Pumpschlauch 30 jeweils zunächst radial in Richtung auf das Pumprad 6 erstreckt und dann in tangentialer Richtung entlang der Anlagefläche 40 bogenförmig umgelenkt wird. Dies begünstigt eine Strömungsrichtung von dem Eingangsschlauch 48 in die Pumpschläuche 36 und aus den Pumpschläuchen 36 in den Austrittsschlauch 44 mit nur wenigen Richtungsänderungen, sodass insgesamt ein geringer Strömungswiderstand erreicht werden kann. In diesem Ausführungsbeispiel erstrecken sich die Pumpschlauchstutzen 36 an dem zweiten Anschlusselement 34 mit ihren Längsachsen in einem Winkel von 90° zu den Längsachsen der Pumpschlauchstutzen 36 an dem ersten Anschlusselement 32, wobei sich die Längsachsen im Wesentlichen im Mittelpunkt bzw. in der Drehachse D des Pumprades 6 schneiden.

Die Schlauchkassette 20 weist in ihrem Inneren eine im Wesentlichen U-förmige Form auf, welche durch die bogenförmige Anlagefläche 40 definiert wird. An den offenen Enden dieser U-förmigen Form erstrecken sich Schenkel 50 schräg nach außen, in denen die Anschlusselemente 32 und 34 angeordnet sind. Die Schenkel 50 erstrecken sich dabei in der Richtung der Pumpschlauchstutzen 36. Die unteren sich schräg zur Einsetzrichtung E erstreckenden und einander zugewandten Seiten der Schenkel 50 bilden Anlageflächen 52, welche an den Gegenanlageflächen 14 des Anlagekörpers 12 anliegen, wenn die Schlauchkassette 20 in die Kassettenaufnahme 10 eingesetzt ist. D.h. die Anlageflächen 52 erstrecken sich im selben Winkel zu der Einsetzrichtung E, wie die Gegenanlageflächen 14.

Das Anschlusselement 32 weist einen Innenraum auf, welcher bis an die Anlagefläche 52 grenzt und im Bereich der Anlagefläche 52 durch eine Membran 54 verschlossen ist. D.h. die Membran 54 erstreckt sich in der Anlagefläche 52 und bildet in diesem Ausführungsbeispiel zumindest näherungsweise 50% der Anlagefläche 52. Die Membran 44 ist somit vom Druck des sich im Inneren des Anschlusselementes 32 befindlichen Fluids beaufschlagt. In einem der Membran 54 zugewandten bzw. gegenüberliegenden Bereich der Gegenanlagefläche 14 ist ein Drucksensor 56 angeordnet. Wenn die Schlauchkassette 20 in die Kassettenaufnahme 10 eingesetzt ist, liegt der Drucksensor 56 an der Membran 54 an und kann auf diese Weise den Druck im Innenraum des Anschlussstutzens 32 erfassen. In diesem Ausführungsbeispiel ist dies der auslassseitige Druck, d.h. der im Austrittsschlauch 44 herrschende Druck. Die Überwachung des austrittsseitigen Drucks kann beispielsweise sinnvoll sein, um einen Überdruck zu erkennen, welcher zum Abspringen des Schlauches oder zum Platzen eines an das Schlauchpumpenaggregat angeschlossenen Auffangbehälters führen könnte. Es ist zu verstehen, dass in entsprechender Weise auch eine Membran 54 mit einem Drucksensor 56 an dem Anschlussstutzen 34 des Eingangsschlauches 48 angeordnet sein könnte.

In dem zweiten Schenkel 50, an welchem der Eingangsschlauch 48 gelegen ist, ist eine Datenspeichereinrichtung 58, beispielsweise in Form eines RFID-Chips, angeordnet bzw. eingebettet. Die Datenspeichereinrichtung 58 kann Informationen über die Art der Schlauchkassette 20 enthalten, welche von einer Steuereinrichtung in dem Motorgehäuse 2 beispielsweise dazu genutzt werden können, den Antriebsmotor in richtiger Weise einzustellen. Alternativ oder zusätzlich könnte beispielsweise auch geprüft werden, ob es sich um eine zugelassene Schlauchkassette 20 für das jeweilige Schlauchpumpen-Aggregat handelt. Zum Lesen und/oder Schreiben der Datenspeichereinrichtung 58 ist eine Leseeinrichtung 60 bzw. Schreib-/Leseeinrichtung 60 in dem Anlagekörper 12 im Bereich der Gegenanlagefläche 14, welche der Datenspeichereinrichtung 58 gegenüberliegt, angeordnet. Die Leseeinrichtung 60 kann beispielsweise eine Antenneneinrichtung sein, welche mit einer geeignet ausgebildeten Steuerelektronik verbunden ist und zum Lesen und/oder Schreiben der Datenspeichereinrichtung 58 ausgestaltet ist. So ist in einem der Schenkel 50 die Membran 54 zur Druckmessung angeordnet, während in dem anderen Schenkel 50 die Datenspeichereinrichtung 58 angeordnet ist. Entsprechend liegt in einer der Gegenanlageflächen 14, derjenigen, die der Membran 54 zugewandt ist, der Drucksensor 56, während in der anderen Gegenanlagefläche 14 die Leseeinrichtung 60 gelegen ist.

### Bezugszeichenliste

- 2: Motorgehäuse
- 3: Welle
- 4: Frontseite
- 6: Pumprad
- 7, 9: Ebene des Pumprades
- 8: Verdrängerrollen
- 10: Kassettenaufnahme
- 12: Anlagekörper
- 14: Gegenanlagefläche
- 16: Halteelemente
- 18: Führungssteg
- 20: Schlauchkassette
- 22: Eingriffsabschnitte
- 23: Ausnehmungen
- 24: Lasche
- 26: Öffnung
- 28: Rastvorsprung
- 30: Pumpschläuche
- 32,34: Anschlusselemente
- 36: Pumpschlauchstutzen
- 40: Anlagefläche
- 42: Anschlussstutzen
- 44: Austrittsschlauch
- 46: Anschlussstutzen
- 48: Eingangsschlauch
- 50: Schenkel
- 52: Anlageflächen
- 54: Membran
- 56: Drucksensor
- 58: Datenspeichereinrichtung
- 60: Leseeinrichtung
- D: Drehachse
- E: Einsetzrichtung

## Patentansprüche

1. Medizinisches Schlauchpumpen-Aggregat mit einem Motorgehäuse (2), welches an seiner Außenseite ein Pumprad (6) aufweist, sowie mit zumindest einer austauschbaren Schlauchkassette (20), in deren Inneren zumindest ein Pumpschlauch (30) angeordnet ist, wobei
das Motorgehäuse (2) an der Außenseite mit dem Pumprad (6) eine Kassettenaufnahme (10) zur Aufnahme der Schlauchkassette (20) aufweist, welche dazu ausgebildet ist, die Schlauchkassette (20) lösbar an dem Motorgehäuse (2) zu fixieren, und wobei die Schlauchkassette (20) und die Kassettenaufnahme (10) derart ausgestaltet sind, dass, wenn die Schlauchkassette (20) an dem Motorgehäuse (2) fixiert ist, der zumindest eine Pumpschlauch (30) mit dem Pumprad (6) in Anlage ist.

2. Medizinisches Schlauchpumpen-Aggregat Anspruch 1, bei welchem die Kassettenaufnahme (10) zumindest ein Halteelement (16) aufweist, welches mit einem Abschnitt der Schlauchkassette (20) formschlüssig in Eingriff ist, wenn die Schlauchkassette (20) in die Kassettenaufnahme (10) eingesetzt ist.

3. Medizinisches Schlauchpumpen-Aggregat nach Anspruch 1 oder 2, bei welchem die Kassettenaufnahme (10) zumindest zwei voneinander beabstandete Haltelemente (16) aufweist und die Schlauchkassette (20) an zwei voneinander abgewandten Seiten Eingriffsabschnitte (22) aufweist, welche jeweils mit einem der Haltelemente (16) in Eingriff ist, wenn die Schlauchkassette (20) in die Kassettenaufnahme (10) eingesetzt ist.

4. Medizinisches Schlauchpumpen-Aggregat nach einem der vorangehenden Ansprüche, bei welchem die zumindest eine Schlauchkassette (20) ein in Einsetzrichtung (E) vorderes Ende aufweist, welches derart offen ausgebildet ist, dass die Schlauchkassette (20) beim Einsetzen in die Kassettenaufnahme (10) mit dem offenen Ende über das Pumprad (6) geschoben werden und dieses übergreifen kann.

5. Medizinisches Schlauchpumpen-Aggregat nach einem der vorangehenden Ansprüche, bei welchem die zumindest eine Schlauchkassette (20) eine offene, dem Motorgehäuse (2) zugewandte Seite aufweist, durch welche sich das Pumprad (6) in das Innere der Schlauchkassette (20) erstreckt, wenn die Schlauchkassette (20) in die Kassettenaufnahme (10) eingesetzt ist.

6. Medizinisches Schlauchpumpen-Aggregat nach einem der vorangehenden Ansprüche, bei welchem die Schlauchkassette (20) eine Trägerstruktur aus Kunststoff aufweist, in welcher der zumindest eine Pumpschlauch (30) an seinen Axialenden gehalten ist, wobei die Schlauchkassette (20) vorzugsweise eine bogenförmige Führung und/oder Anlagefläche (40) für den zumindest einen Pumpschlauch (30) aufweist.

7. Medizinisches Schlauchpumpen-Aggregat nach einem der vorangehenden Ansprüche, bei welchem die Schlauchkassette (20) zumindest ein, vorzugsweise zwei, Anschlusselemente (32, 34) aufweist, welche den zumindest einen Pumpschlauch (30) mit einem Eingangsschlauch (48) und/oder einem Austrittsschlauch (44) verbinden, wobei vorzugsweise die Anschlusselemente (32, 34) aus Kunststoff gefertigt sind und weiter bevorzugt einstückig mit der Trägerstruktur des Schlauchkassette (20) ausgebildet sind oder in der Trägerstruktur kraft- und/oder formschlüssig fixiert sind.

8. Medizinisches Schlauchpumpen-Aggregat nach einem der vorangehenden Ansprüche, bei welchem die Schlauchkassette (20) mit dem zumindest einen Pumpschlauch (30) als austauschbarer zum Einmalgebrauch vorgesehener Artikel ausgebildet ist.

9. Medizinisches Schlauchpumpen-Aggregat nach einem der vorangehenden Ansprüche, bei welchem die Schlauchkassette (20) in ihrem Inneren zwei parallel zueinander verlaufende Pumpschläuche (30) aufweist und das Pumprad (6) so ausgebildet ist, dass es mit beiden Pumpschläuchen (30) in Anlage ist, wenn die Schlauchkassette (20) in die Kassettenaufnahme (10) eingesetzt ist.

10. Medizinisches Schlauchpumpen-Aggregat nach einem der vorangehenden Ansprüche, bei welchem das Pumprad (6) zumindest zwei Verdrängerkörper (8) aufweist, von welchen zumindest ein erster Verdrängerkörper (8) auf einen ersten der beiden parallel zueinander verlaufenden Pumpschläuche (30) einwirkt und zumindest ein zweiter Verdrängerkörper (8) auf einen zweiten der beiden parallel zueinander verlaufenden Pumpschläuche (30) einwirkt, wobei vorzugsweise der zumindest eine erste und der zumindest eine zweite Verdrängerkörper (8) in Drehrichtung versetzt an dem Pumprad (6) angeordnet sind.

11. Medizinisches Schlauchpumpen-Aggregat nach einem der vorangehenden Ansprüche, bei welchem die Anschlusselemente (32, 34) jeweils zumindest einen, vorzugsweise zwei, Pumpschlauchstutzen (36) zum Anschluss jeweils eines Pumpschlauches (30) und einen Anschlussstutzen (46, 42) zum Anschluss eines Eingangs- (48) oder Austrittsschlauches (44) aufweisen, wobei sich die Pumpschlauchstutzen (36) und die Anschlussstutzen (46,42) von dem Anschlusselement (32, 34) vorzugsweise in entgegengesetzte Richtungen erstrecken.

12. Medizinisches Schlauchpumpen-Aggregat nach einem der vorangehenden Ansprüche, bei welcher zumindest eines der Anschlusselemente (32, 34), vorzugsweise das zur Verbindung mit einem Austrittsschlauch (44) vorgesehene Anschlusselement (32), zur Aufnahme eines Drucksensors (56) oder zur Anlage an einem Drucksensor (56) ausgebildet ist.

13. Medizinisches Schlauchpumpen-Aggregat nach einem der vorangehenden Ansprüche, bei welchem eines der Anschlusselemente (32, 34), vorzugsweise das zur Verbindung mit einem Austrittsschlauch (44) vorgesehene Anschlusselement (32), eine an einen Fluidkanal im Inneren des Anschlusselementes (32, 34) angrenzende Membran (54) aufweist und die Kassettenaufnahme (10) einen Sensor (56) aufweist, welcher derart angeordnet und ausgebildet ist, dass er mit der Membran (54) zur Druckmessung zusammenwirkt, wenn die Schlauchkassette (20) in die Kassettenaufnahme (10) eingesetzt ist.

14. Medizinisches Schlauchpumpen-Aggregat nach einem der vorangehenden Ansprüche, bei welchem die zumindest eine Schlauchkassette (20) an ihrem in Einsetzrichtung (E) vorderen Ende zumindest eine sich quer zu der Einsetzrichtung (E), vorzugsweise schräg zur Einsetzrichtung (E) erstreckendende Anlagefläche (52)aufweist und die Kassettenaufnahme (10) zumindest eine Gegenanlagefläche (14) aufweist, welche so angeordnet ist, dass sie der Anlagefläche (52) gegenüberliegt und vorzugsweise an der Anlagefläche (52) anliegt, wenn die Schlauchkassette (20) in die Kassettenaufnahme (10) eingesetzt ist, wobei vorzugsweise zumindest eine an einen Fluidkanal der Schlauchkassette (20) angrenzende Membran (54) in einer Anlagefläche (52) gelegen ist und zumindest ein mit der Membran (54) zusammenwirkenden Sensor (56) in einer gegenüberliegenden Gegenanlagefläche (14) angeordnet ist.

15. Medizinisches Schlauchpumpen-Aggregat nach Anspruch 14, bei welchem zumindest ein Datenspeicherelement an einer Anlagefläche (52) und eine zum Auslesen der Datenspeichereinrichtung (58) ausgebildete Leseeinrichtung (60) an einer gegenüberliegenden Gegenanlagefläche (14) gelegen ist.
